# DEMANDE DE BREVET EUROPEEN

(11) **EP 1 366 749 A1**
(43) Date de publication de la demande: **03.12.2003**
(21) Numéro de dépôt: 03291141.4
(22) Date de dépôt: 15.05.2003
(51) Int. Cl.: A61K 7/11, C09K 3/30, C08L 53/00, B65D 83/16, B65D 83/14

(54) **Dispositif aérosol à deux compartiments comprenant au moins un polymère linéaire séquencé amphiphile**

(30) Priorité: 31.05.2002 FR 0206737
(71) Demandeur: L'OREAL, 75008 Paris (FR)
(72) Inventeur: Dubief, Claude, 78150 Le Chesnay (FR); Giroud, Franck, 92110 Clichy (FR); Rollat-Corvol, Isabelle, 75017 Paris (FR)
(74) Mandataire: Bourdeau, Françoise

(57) **Abrégé**

La présente invention concerne un dispositif aérosol à deux compartiments, comprenant :
- dans un premier compartiment, une composition de coiffage qui comprend au moins un copolymère linéaire séquencé, comprenant au moins un bloc hydrophile et au moins un bloc hydrophobe, le ou les blocs hydrophiles représentant au moins 30 % en poids du copolymère linéaire séquencé, à l'exclusion des copolymères séquencés d'oxyde d'éthylène et d'oxyde de propylène, des copolymères séquencés à motifs uréthanne et des copolymères séquencés à motifs siloxane, et
- dans un second compartiment, un gaz comprimé.

## Description

La présente invention est relative à un dispositif aérosol à deux compartiments comprenant une composition de coiffage contenant au moins un polymère séquencé amphiphile et un gaz comprimé, et à un procédé de coiffage.

Les compositions de coiffage, telles que les laques et les sprays, conditionnées sous forme de spray aérosol sont généralement composées d'une phase liquide comprenant, dans un milieu cosmétiquement acceptable alcoolique ou hydroalcoolique, au moins un polymère filmogène, et d'un agent propulseur qui est un gaz liquéfié sous pression réduite ou dissous dans la phase liquide.

Dans le domaine des produits capillaires, on cherche à fabriquer des laques en aérosol ne comportant aucun composé organique volatil tel que l'éthanol ou le diméthyléther, et ce pour des raisons essentiellement écologiques, tout en conservant de bonnes propriétés de mise en forme et de maintien de la coiffure.
Le document US 5 626 840 décrit des compositions de fixation des cheveux qui peuvent se trouver sous la forme d'aérosol. Elles comprennent un polyuréthane dans un milieu aqueux ou hydroalcoolique, une base minérale ou organique, et un solvant qui peut être de l'eau ou un mélange d'eau et de solvant organique polaire.
Ce document décrit une nette diminution des composés organiques volatils dans les compositions de coiffage.
La Demanderesse a découvert, de manière surprenante, que l'utilisation d'un dispositif à deux compartiments, comprenant dans un premier compartiment une composition de coiffage contenant au moins un copolymère linéaire séquence, comprenant au moins un bloc hydrophile et au moins un bloc hydrophobe, le ou les blocs hydrophiles représentant au moins 30 % en poids du copolymère linéaire séquencé, à l'exclusion des copolymères séquencés d'oxyde d'éthylène et d'oxyde de propylène, des copolymères séquencés à motifs uréthanne et des copolymères séquencés à motifs siloxane, et dans un second compartiment un gaz comprimé, permettait d'obtenir une laque présentant des propriétés de mise en forme et de maintien tout au moins équivalentes à celles des laques de l'état de la technique et ne comportant en outre aucun composé organique volatil.
Lesdites propriétés sont obtenues en particulier lorsque la composition de coiffage comprend au moins un copolymère linéaire séquencé, comprenant au moins un bloc hydrophile et au moins un bloc hydrophobe, le ou les blocs hydrophiles représentant au moins 30 % en poids du copolymère linéaire séquencé, à l'exclusion des copolymères séquencés d'oxyde d'éthylène et d'oxyde de propylène, des copolymères séquencés à motifs uréthanne et des copolymères séquencés à motifs siloxane.

La présente invention a donc pour objet un dispositif aérosol à deux compartiments comprenant,
- dans un premier compartiment, une composition de coiffage qui comprend au moins un copolymère linéaire séquencé, comprenant au moins un bloc hydrophile et au moins un bloc hydrophobe, le ou les blocs hydrophiles représentant au moins 30 % en poids du copolymère linéaire séquencé, à l'exclusion des copolymères séquencés d'oxyde d'éthylène et d'oxyde de propylène, des copolymères séquencés à motifs uréthanne et des copolymères séquencés à motifs siloxane, et
- dans un second compartiment, un gaz comprimé.

Un autre objet de la présente invention consiste en un procédé de coiffage mettant en oeuvre le dispositif de l'invention.
D'autres objets, caractéristiques, aspects et avantages de l'invention apparaîtront encore plus clairement à la lecture de la description et des divers exemples qui suivent.

De préférence, le gaz comprimé est choisi parmi l'air, l'azote, le gaz carbonique et leurs mélanges, l'air étant particulièrement préféré.

Ledit gaz comprimé est utilisé de préférence sous une pression comprise entre 1 et 12 bars, mieux encore comprise entre 9 et 11 bars.

Les copolymères linéaires séquencés utilisables selon la présente invention sont des copolymères dits "amphiphiles", à savoir des copolymères comportant à la fois des blocs hydrophobes et des blocs hydrophiles.
On entend par blocs hydrophobes selon la présente invention des blocs comprenant au moins 75 % en moles de monomères insolubles dans l'eau et par blocs hydrophiles des blocs comprenant au moins 75 % en moles de monomères hydrosolubles.

Les monomères hydrosolubles formant les blocs hydrophiles des copolymères séquencés utilisés dans la présente invention peuvent être de nature anionique, non-ionique ou cationique et peuvent être utilisés seuls ou sous forme de mélange contenant deux ou plusieurs monomères différents.

On peut citer à titre d'exemples de monomères hydrosolubles anioniques, les acides carboxyliques à insaturation éthylénique, tels que l'acide acrylique, l'acide méthacrylique, l'acide itaconique, l'acide fumarique, l'acide crotonique et l'acide maléique, l'acide 2-acrylamido-2-méthylpropanesulfonique, l'acide styrènesulfonique, l'acide vinylsulfonique et l'acide vinylphosphonique.
Les monomères hydrosolubles non-ioniques englobent, entre autres, l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate d'hydroxypropyle.
Enfin, les monomères hydrosolubles cationiques englobent par exemple le chlorure de diméthyldiallylammonium, le chlorure de méthylvinylimidazolium, la 2-vinylpyridine, la 4-vinylpyridine, la 2-méthyl-5-vinylpyridine, la vinylamine, les monomères de formule

H₂C=CR₁-CO-X₂

dans laquelle
R₁ représente un atome d'hydrogène ou un groupe méthyle,
X₂ représente un groupe hydrocarboné linéaire ou ramifié en C₁₋₆ portant au moins une fonction amine primaire, secondaire, tertiaire ou au moins un atome d'azote quaternaire, ou un groupe de formule NHR₂ ou de formule NR₂R₃ où R₂ et R₃ représentent indépendamment l'un de l'autre chacun un groupe hydrocarboné en C₁₋ 6, linéaire ou ramifié, portant au moins une fonction amine primaire, secondaire, tertiaire ou au moins un atome d'azote quaternaire.

Les monomères insolubles dans l'eau formant les blocs hydrophobes des copolymères séquencés sont choisis de préférence parmi les monomères vinylaromatiques tels que le styrène et ses dérivés alkylés comme le 4-butylstyrène, l'□-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C₁₋₁₀, d'aryle en C₆₋₁₀ ou d'aralkyle en C₆₋₁₀ et les méthacrylates d'alkyle en C₁₋₁₀, d'aryle en C₆₋₁₀ ou d'aralkyle en C₆₋₁₀, comme par exemple les (meth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les fluoroacrylates d'alkyle.

Comme indiqué ci-dessus à propos de la définition des blocs hydrophobes et hydrophiles des copolymères séquencés, les monomères insolubles dans l'eau et les monomères hydrosolubles représentent au moins 75 % en moles respectivement des blocs hydrophobes et hydrophiles. Autrement dit, chaque bloc hydrophobe peut comprendre jusqu'à 25 % en moles d'un ou de plusieurs monomères hydrosolubles. Cette proportion est de préférence au plus égale 10 % en moles et idéalement inférieure ou égale à 5 % en moles.
De manière analogue, chaque bloc hydrophile peut comprendre jusqu'à 25 % en moles, de préférence jusqu'à 10 % en moles, et idéalement jusqu'à 5 % en moles, d'un ou de plusieurs monomères insolubles dans l'eau.
Les copolymères linéaires séquencés utilisés englobent bien entendu également ceux dans lesquels les blocs hydrophiles et les blocs hydrophobes sont constitués exclusivement respectivement de monomères hydrosolubles et de monomères insolubles dans l'eau. Ces blocs peuvent être des blocs homopolymères ou des blocs copolymères renfermant deux ou plus de deux monomères différents du même type.
De préférence, les copolymères linéaires séquencés de l'invention sont hydrosolubles ou dispersibles dans l'eau et plus préférentiellement encore, ils sont hydrosolubles.

On entend par monomère ou polymère hydrosoluble un monomère ou polymère qui, lorsqu'on l'introduit dans de l'eau à une température de 25 °C, et à une concentration en poids égale à 0,5 %, permet l'obtention d'une solution macroscopiquement homogène et transparente, c'est-à-dire ayant une valeur de transmittance de la lumière, à une longueur d'onde égale à 500 nm, à travers un échantillon de 1 cm d'épaisseur, d'au moins 70 %, de préférence d'au moins 80 %.
Les copolymères dispersibles dans l'eau selon l'invention peuvent se présenter sous forme de latex ou de pseudo latex.
De préférence, les copolymères linéaires séquencés selon l'invention sont hydrosolubles.
Cette aptitude à être dissous ou finement dispersé dans l'eau est liée à la proportion importante des séquences hydrophiles dans les copolymères séquencés amphiphiles.
Cette proportion doit être d'au moins 30 % en poids, et elle est de préférence supérieure ou égale à 60 % en poids, sans pour autant dépasser 97 % en poids, de préférence 95 % en poids.

La masse moléculaire moyenne en nombre de chaque bloc, qu'il soit hydrophobe ou hydrophile, copolymère ou homopolymère, est de préférence comprise entre 500 et 100 000, en particulier entre 500 et 50 000, avec un indice de polydispersité (M_{w}/Mₙ) compris entre 1,01 et 3,0, de préférence entre 1,1 et 2,5.

Les copolymères linéaires séquencés utilisés dans la présente invention peuvent être
- des copolymères diblocs de formule AB,
- des copolymères triblocs de formule ABA ou BAB et
- des copolymères multiblocs comprenant, au moins deux blocs hydrophiles et au moins deux blocs hydrophobes disposés en alternance, chaque A représentant un bloc hydrophile et chaque B représentant un bloc hydrophobe, les blocs A d'un même polymère pouvant être identiques ou différents et les blocs B d'un même polymère pouvant être identiques ou différents.

On préfère en particulier des copolymères diblocs et des copolymères triblocs comportant un bloc central hydrophile et deux blocs latéraux hydrophobes.

Les polymères blocs de l'invention peuvent être préparés par les procédés de synthèse classiquement utilisés pour obtenir des polymères blocs. On peut citer par exemple les polymérisations anionique ou cationique, et la polymérisation radicalaire contrôlée (voir *"New Method* of Polymer *Synthesis",* Blackie Academic & Professional, Londres, 1995, volume 2, page 1, ou Trends Polym. Sci. 4, page 183 (1996) de C. J. Hawker), qui peut être mise en oeuvre suivant différents procédés comme par exemple la polymérisation radicalaire par transfert d'atomes *(Atom Transfert Radical Polymerization* ou ATRP) (voir *JACS,* 117, page 5614 (1995), de Matyjasezwski *et al.),* la méthode des radicaux tels que les nitroxydes (Georges et al., Macromolecules, 1993, 26, 2987).
On peut aussi utiliser ces procédés pour obtenir un seul des deux types de blocs du polymère de l'invention, l'autre bloc étant introduit dans le polymère final par l'intermédiaire de l'amorceur utilisé ou bien par réaction de couplage entre les blocs hydrophiles et hydrophobes.

La quantité de copolymères linéaires séquencés amphiphiles dans les compositions de la présente invention dépend d'un grand nombre de paramètres parmi lesquels on peut citer la masse moléculaire des copolymères, le nombre et de la taille des blocs hydrophiles et hydrophobes, la quantité de polymères bénéfiques pour la chevelure, et surtout la viscosité de la composition que l'on souhaite obtenir.
On obtient généralement des résultats satisfaisants avec une quantité de copolymères linéaires séquencés comprise entre 0,01 et 10 % en poids, de préférence entre 0,1 et 5 % en poids rapporté au poids de la composition capillaire.

La composition de coiffage contenue dans le dispositif selon l'invention présente avantageusement une teneur en eau comprise entre 30 et 99,9 % en poids, de préférence entre 50 et 99 % en poids, et encore plus préférentiellement entre 75 et 98 % par rapport au poids total de ladite composition.
La composition de coiffage peut comprendre en outre des additifs tels que des silicones sous forme soluble, dispersée, micro-dispersée, des actifs traitants, des hydratants comme le glycérol, des filtres UV, des acides, des bases, des agents plastifiants, des agents solubilisants, des agents conservateurs, des vitamines et des provitamines, des colorants, des pigments, des agents tensioactifs anioniques, cationiques, non ioniques ou amphotères, des parfums, des agents anti-corrosion, et leurs mélanges.
L'homme de métier veillera à choisir les éventuels additifs et leur quantité de manière à ce qu'ils ne nuisent pas aux propriétés des compositions de la présente invention.

Ces additifs sont notamment présents dans la composition selon l'invention en une quantité allant de 0 à 20 % en poids par rapport au poids total de la composition.
De préférence, le dispositif aérosol à deux compartiments est constitué par un bidon aérosol externe comportant une poche interne soudée hermétiquement à une valve. La composition est introduite dans la poche interne et un gaz comprimé est introduit entre 1a poche et le bidon à une pression suffisante pour faire sortir le produit sous forme d'un spray à travers l'orifice d'une buse. Un tel dispositif est commercialisé sous le nom EP SPRAY par la société EP-SPRAY SYSTEM SA.
Les dispositifs aérosols de l'invention sont de préférence des laques pour cheveux.
La présente invention concerne également un procédé de coiffage qui consiste en ce que l'on vaporise la composition de coiffage contenue dans le dispositif aérosol selon l'invention, sur les cheveux mouillés ou non.
Les exemples suivants sont donnés à titre illustratif de la présente invention.

### Exemple 1

On a préparé une composition de coiffage à partir des ingrédients suivants :

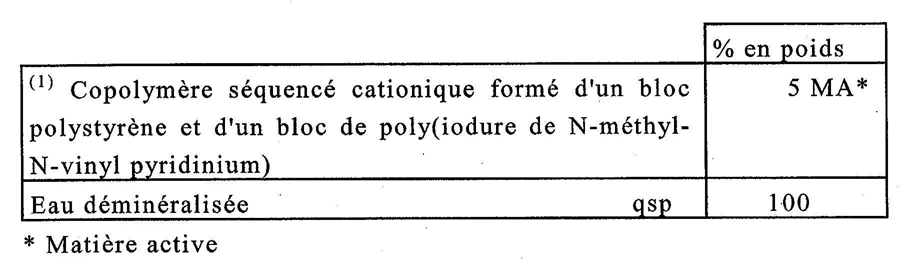

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonnaire.

La poche est remplie avec la composition comme indiqué ci-dessus. De l'air comprimé est introduit entre la poche et le bidon.
On a vaporisé la composition sur des cheveux secs. La pulvérisation se fait sous forme d'un spray doux.
On obtient après séchage, une chevelure présentant un bon maintien.

## Revendications

1. Dispositif aérosol à deux compartiments, comprenant :
- dans un premier compartiment, une composition de coiffage qui comprend au moins un copolymère linéaire séquencé, comprenant au moins un bloc hydrophile et au moins un bloc hydrophobe, le ou les blocs hydrophiles représentant au moins 30 % en poids du copolymère linéaire séquencé, à l'exclusion des copolymères séquencés d'oxyde d'éthylène et d'oxyde de propylène, des copolymères séquencés à motifs uréthanne et des copolymères séquencés à motifs siloxane, et
- dans un second compartiment, un gaz comprimé.

2. Dispositif aérosol selon la revendication 1, **caractérisé en ce que** le gaz comprimé est choisi parmi l'air, l'azote, le gaz carbonique et leurs mélanges, l'air étant particulièrement préféré.

3. Dispositif aérosol selon la revendication 1 ou 2, **caractérisé en ce que** la pression du gaz comprimé est comprise entre 1 et 12 bars.

4. Dispositif aérosol selon la revendication 3, **caractérisé en ce que** la pression du gaz comprimé est comprise entre 9 et 11 bars.

5. Dispositif aérosol selon l'une des revendications précédentes, **caractérisé par le fait que** le copolymère linéaire séquencé est choisi parmi les copolymères diblocs de formule AB, les copolymères triblocs de formule ABA ou BAB et les copolymères multiblocs comprenant, au moins deux blocs hydrophiles et au moins deux blocs hydrophobes disposés en alternance, chaque A représentant un bloc hydrophile et chaque B représentant un bloc hydrophobe, les blocs A d'un même polymère pouvant être identiques ou différents et les blocs B d'un même polymère pouvant être identiques ou différents.

6. Dispositif aérosol selon la revendication 5, **caractérisé par le fait que** le copolymère linéaire séquencé est choisi parmi les copolymères diblocs et les copolymères triblocs comportant un bloc central hydrophile et deux blocs latéraux hydrophobes.

7. Dispositif aérosol selon l'une quelconque des revendications précédentes, **caractérisé par le fait que** les blocs hydrophiles sont formés de monomères hydrosolubles choisis parmi les monomères hydrosolubles anioniques, les monomères hydrosolubles non-ioniques et les monomères hydrosolubles cationiques ou un mélange de ceux-ci.

8. Dispositif aérosol selon la revendication 7, **caractérisé par le fait que** les monomères hydrosolubles anioniques sont choisis parmi les acides carboxyliques à insaturation éthylénique, l'acide 2-acrylamido-2-méthylpropanesulfonique, l'acide styrènesulfonique, l'acide vinylsulfonique et l'acide vinylphosphonique.

9. Dispositif aérosol selon la revendication 7, **caractérisé par le fait que** les monomères hydrosolubles non-ioniques sont choisis parmi l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique, l'oxyde d'éthylène, l'acrylate d'hydroxyéthyle, l'acrylate d'hydroxypropyle, le méthacrylate d'hydroxyéthyle et le méthacrylate d'hydroxypropyle.

10. Dispositif aérosol selon la revendication 7, **caractérisé par le fait que** les monomères hydrosolubles cationiques sont choisis parmi le chlorure de diméthyldiallylammonium, le chlorure de méthylvinylimidazolium, la 2-vinylpyridine, la 4-vinylpyridine, la 2-méthyl-5-vinylpyridine, la vinylamine, les monomères de formule
H₂C=CR₁-CO-X₂
dans laquelle
R₁ représente un atome d'hydrogène ou un groupe méthyle,
X₂ représente un groupe hydrocarboné linéaire ou ramifié en C₁₋₆ portant au moins une fonction amine primaire, secondaire, tertiaire ou au moins un atome d'azote quaternaire, ou un groupe de formule NHR₂ ou de formule NR₂R₃ où R₂ et R₃ représentent indépendamment l'un de l'autre chacun un groupe hydrocarboné en C₁₋₆, linéaire ou ramifié, portant au moins une fonction amine primaire, secondaire, tertiaire ou au moins un atome d'azote quaternaire.

11. Dispositif aérosol selon l'une quelconque des revendications précédentes, **caractérisé par le fait que** les blocs hydrophobes sont formés de monomères insolubles dans l'eau choisis parmi les monomères vinylaromatiques, les diènes et les dérivés alkylés des diènes, le chloroprène, les acrylates d'alkyle en C_{1-10,} d'aryle en C₆₋₁₀ ou d'aralkyle en C_{1-10,} les méthacrylates d'alkyle en C_{1-10,} d'aryle en C₆₋₁₀ ou d'aralkyle en C_{1-10,} l'acétate de vinyle, les vinyléthers de formule CH₂=CH-O-R et les allyléthers de formule CH₂=CH-CH₂-O-R où R représente un groupe alkyle en C_{1-6,} l'acrylonitrile, le chlorure de vinyle, le chlorure de vinylidène, la caprolactone, l'éthylène, le propylène et les monomères vinyliques fluorés ou à chaîne perfluorée.

12. Dispositif aérosol selon l'une quelconque des revendications 7 à 10, **caractérisé par le fait que** les blocs hydrophiles contiennent jusqu'à 25 % en moles, de préférence jusqu'à 10 % en moles, et idéalement jusqu'à 5 % en moles, d'un ou de plusieurs monomères non solubles dans l'eau selon la revendication 11.

13. Dispositif aérosol selon la revendication 11, **caractérisée par le fait que** le ou les blocs hydrophobes contiennent jusqu'à 25 % en moles, de préférence jusqu'à 10 % en moles, et idéalement jusqu'à 5 % en moles, d'un ou de plusieurs monomères hydrosolubles selon l'une quelconque des revendications 8 à 10.

14. Dispositif aérosol selon l'une quelconque des revendications précédentes, **caractérisé par le fait que** le ou les copolymères séquencés sont hydrosolubles ou dispersibles dans l'eau.

15. Dispositif aérosol selon la revendication 14, **caractérisé par le fait que** 1e ou les copolymères séquencés sont hydrosolubles.

16. Dispositif aérosol selon l'une quelconque des revendications précédentes, **caractérisé par le fait que** le ou les blocs hydrophiles représentent au moins 30 % en poids du copolymère linéaire séquencé.

17. Composition capillaire selon l'une quelconque des revendications précédentes, **caractérisée par le fait que** le ou les copolymères linéaires séquencés sont présents à raison de 0,01 à 10 % en poids, de préférence à raison de 0,1 à 5 % en poids rapporté à la composition capillaire.

18. Dispositif aérosol selon l'une quelconque des revendications précédentes, **caractérisé en ce que** la composition de coiffage présente une teneur en eau comprise 30 et 99,9 % en poids, de préférence entre 50 et 99 % en poids, et encore plus préférentiellement entre 75 et 98 % par rapport au poids total de ladite composition.

19. Dispositif aérosol selon l'une quelconque des revendications précédentes, **caractérisé en ce que** la composition de coiffage comprend en outre des additifs choisis parmi des silicones sous forme soluble, dispersée, micro-dispersée, des actifs traitants, des hydratants, des filtres UV, des acides, des bases, des agents plastifiants, des agents solubilisants, des agents conservateurs, des vitamines et des provitamines, des colorants, des pigments, des agents tensioactifs anioniques, cationiques, non ioniques ou amphotères, des parfums, des agents anti-corrosion, et leurs mélanges.

20. Dispositif aérosol selon l'une quelconque des revendications précédentes, **caractérisé en ce qu'**il constitue une laque pour cheveux.

21. Procédé de coiffage, **caractérisé en ce que** l'on vaporise la composition de coiffage contenue dans le dispositif aérosol selon l'invention, sur les cheveux mouillés ou non.
